# EUROPEAN PATENT SPECIFICATION

(11) **EP 3 840 742 B1**
(45) Date of publication and mention of the grant of the patent: **24.04.2024**
(21) Application number: 19846183.2
(22) Date of filing: 08.08.2019
(51) Int. Cl.: A61K 31/198, A61K 31/352, A61P 25/00, C07C 229/24, C07D 311/80

(54) **L-THEANINE AGAINST THC-INDUCED EFFECTS**
L-THEANIN GEGEN THC-INDUZIERTE WIRKUNGEN
L-THÉANINE CONTRE LES EFFETS INDUITS PAR LE THC

(30) Priority: 08.08.2018 US 201862715828 P; 12.09.2018 US 201862730253 P
(43) Date of publication of application: 30.06.2021
(73) Proprietor: Laviolette, Steven Robert, London ON N6C 3N1 (CA); De Felice, Marta, London ON N6C 1L7 (CA); Rusiniak, Richard, Mississauga ON L5L 5S9 (CA); Ramsay, Paul, Mississauga ON L5L 5S9 (CA)
(72) Inventor: Laviolette, Steven Robert, London ON N6C 3N1 (CA); De Felice, Marta, London ON N6C 1L7 (CA); Rusiniak, Richard, Mississauga ON L5L 5S9 (CA); Ramsay, Paul, Mississauga ON L5L 5S9 (CA)
(74) Representative: Uexküll & Stolberg
(86) International application number: PCT/CA2019/051084
(87) International publication number: WO 2020/028988

(56) References cited:
- CA-A1- 3 013 573
- US-A1- 2015 290 181
- US-A1- 2016 038 509
- US-A1- 2016 213 624
- US-A1- 2016 213 624
- Anonymous: "A Post-Weed Tonic to Make You Less High, Reviewed", Eloise LeBel MEL Magazine, 10 March 2017 (2017-03-10), pages 1-4, XP055801795, Retrieved from the Internet: URL:https://melmagazine.com/en-us/story/a- post-weed-tonic-to-make-vou-less-high-revi ewed [retrieved on 2021-05-06]
- Ace: "Forum online", Grasscity forums, 19 September 2011 (2011-09-19), XP055916128, Retrieved from the Internet: URL:https://forum.grasscity.com/threads/l- theanine-supplements-and-weed.908748/
- Anonymous: "Meet 1906, Our Vendor of the Month! -", Green Man Cannabis, 1 June 2017 (2017-06-01), XP055685107, Retrieved from the Internet: URL:https://www.greenmancannabis.com/2017/ 06/meet-1906-vendor-month/ [retrieved on 2020-04-14]
- Anonymous: "Marijuana Review: ZootDrops Relaxation Blend", FISCHER CAMPBELL HIGH ABOVE SEATTLE WEBSITE,, 12 March 2015 (2015-03-12), XP055685110, Retrieved from the Internet: URL:http://highaboveseattle.com/marijuana- review-zootdrops-relaxation-blend/ [retrieved on 2020-04-14]
- RENARD JUSTINE ET AL: "Adolescent THC Exposure Causes Enduring Prefrontal Cortical Disruption of GABAergic Inhibition and Dysregulation of Sub-Cortical Dopamine Function", SCIENTIFIC REPORTS, vol. 7, no. 1, 12 September 2017 (2017-09-12), pages 1-14, XP055916142, DOI: 10.1038/s41598-017-11645-8 Retrieved from the Internet: URL:https://www.nature.com/articles/s41598 -017-11645-8.pdf>
- MICHAEL S. RITSNER ET AL: "L-Theanine Relieves Positive, Activation, and Anxiety Symptoms in Patients With Schizophrenia and Schizoaffective Disorder : An 8-Week, Randomized, Double-Blind, Placebo-Controlled, 2-Center Study", JOURNAL OF CLINICAL PSYCHIATRY, vol. 72, no. 01, 30 November 2010 (2010-11-30), pages 34-42, XP055626284, US ISSN: 0160-6689, DOI: 10.4088/JCP.09m05324gre
- "A Post-Weed Tonic to Make You Less High, Reviewed", Eloise LeBel MEL Magazine, 10 March 2017 (2017-03-10), page 4, XP055801795, Retrieved from the Internet: URL:https://melmagazine.com/en-us/story/a- post-weed-tonic-to-make-vou-less-high-revi ewed
- Organa Brands: "Introducing our newest brand. Pressies these functionally enhanced cannabis tablets", Facebook Facebook, 19 June 2018 (2018-06-19), page 1, XP009527453, Retrieved from the Internet: URL:https://www.facebook.com/organabrands/ posts/introducing-our-newest-brand-pressie s-these-functionally-enhanced-cannabis-tab lets/604723409900791
- "Meet 1906, Our Vendor of the Month!", Green Man Cannabis website, 14 June 2017 (2017-06-14), page 3, XP055685107, Retrieved from the Internet: URL:https://www.greenmancannabis.com/2017/ 06/meet-1906-vendor-month/
- "Marijuana Review: ZootDrops Relaxation Blend", Fischer Campbell High Above Seattle Website, 12 March 2015 (2015-03-12), page 5, XP055685110, Retrieved from the Internet: URL:http://highaboveseattle.com/marijuana- review-zootdrops-relaxation-blend/
- REALINI N ET AL: "Chronic URB597 treatment at adulthood reverted most depressive-like symptoms induced by adolescent exposure to THC in female rats", NEUROPHARMACOLOGY, ELSEVIER, AMSTERDAM, NL, vol. 60, no. 2-3, 1 February 2011 (2011-02-01), pages 235-243, XP027579444, ISSN: 0028-3908 [retrieved on 2010-12-29]

## Description

### FIELD OF THE INVENTION

The invention relates to the field of medical marijuana.

### BACKGROUND

The use of marijuana is known to cause neuronal, molecular and neuropsychiatric side effects. This complicates the use of marijuana for medical treatment. Δ-9-tetrahydrocannabinol (THC) is the primary psychoactive compound in marijuana (MJ). As demonstrated in both clinical and pre-clinical research, THC is associated with a wide-range of deleterious neuropsychiatric side-effects in both adolescent and adult users of MJ. In particular, chronic or acute THC exposure has been shown to increase anxiety, increase depressive symptoms, increase vulnerability to schizophrenia-related symptoms and to produce profound cognitive side effects such as deficits in cognitive filtering and memory impairments. Given the increasing use of 'medical' marijuana for the treatment of wide variety of ailments, there is therefore an urgent need for the development of a safer THC formulation that lacks the neurocognitive and neuropsychiatric side-effects associated with MJ usage. In addition, marijuana and specific THC derivatives are now widely used for a variety of symptoms and disease states, including pain management, control of chemotherapy-related nausea, glaucoma, multiple sclerosis and others.

RENARD JUSTINE ET AL: "Adolescent THC Exposure Causes Enduring Prefrontal Cortical Disruption of GABAergic Inhibition and Dysregulation of Sub-Cortical Dopamine Function", SCIENTIFIC REPORTS, Vol. 7, No. 1, pages 1 -14 states that chronic adolescent marijuana use has been linked to the later development of psychiatric diseases such as schizophrenia and studied exposure of adolescent rats to Δ-9-tetra-hydrocannabinol (THC), the psychoactive component in marijuana. REALINI N ET AL: "Chronic URB597 treatment at adulthood reverted most depressive-like symptoms induced by adolescent exposure to THC in female rats", NEUROPHARMACOLOGY, ELSEVIER, AMSTERDAM, NL, vol. 60, no. 2-3, pages 235-243 describes depressive-like symptoms present in these female rats. Adult female rats pretreated with THC display passive coping strategy towards acute stressful situations as demonstrated by their behaviours in the first session of the forced swim test, develop a profound anhedonic state as demonstrated by the reduced consumption of palatable food and present a decrease in social functioning.

Importantly, one of the primary concerns (from patients and physicians) associated with therapeutic marijuana use and prescription is the neuropsychiatric side-effects associated with THC.

### SUMMARY

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

The invention relates to L-Theanine for use in a method of treatment for blocking THC-induced anhedonic, depressive-like effects, wherein the treatment regime includes the exposure of a patient to Δ-9-Tetrahydrocannabinol (THC).

Preferred embodiments are specified in claims 2 to 4.

According to one aspect, the method of administration can be such that the peak bioavailability of THC occurs substantially contemporaneously with the peak bioavailability of Theanine.

According to another aspect, the administration can be by needle, transdermal, ingestion, inhalation, sublingual or by suppository.

A consumer package adapted for administering Theanine can be selected from the group consisting of: IV bag, prefilled syringe, cream, patch, pill, capsule, liquid, inhaler, sublingual strip, suppository and edible.

Advantages, features and characteristics of the invention will become evident to persons of ordinary skill in the art upon review of the following description, with reference to the appended drawings, the latter being briefly described hereinafter.

### BRIEF DESCRIPTION OF THE DRAWINGS

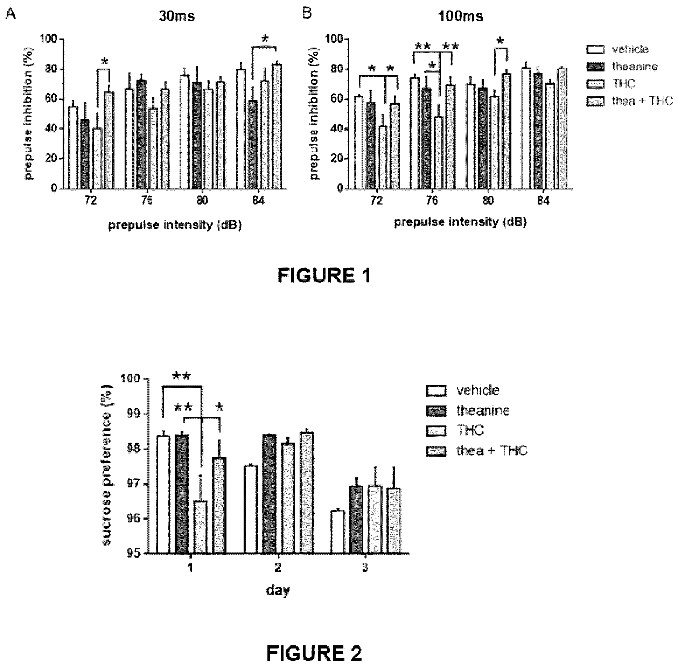

FIG. 1A. Using a pre-clinical model of cognitive filtering in rats (paired-pulse-inhibition) it was demonstrated that L-Theanine (10 mg/kg) co-administration with THC blocked the effects of THC alone on the disruption of sensorimotor filtering at 30 msec stimulus intervals at 72 and 84 decibel stimulus intensities.
FIG. 1B. Using a pre-clinical model of cognitive filtering in rats (paired-pulse-inhibition) it was demonstrated that L-Theanine (10 mg/kg) co-administration with THC blocked the effects of THC alone on the disruption of sensorimotor filtering at 100 msec stimulus intervals at 72, 76 and 80 decibel stimulus intensities.
FIG. 2. Using a pre-clinical model of depression (sucrose preference task) in rats, it was demonstrated that L-Theanine (10 mg/kg) co-administration with THC, reversed the anhedonic (depression-like) effects of chronic THC administration.

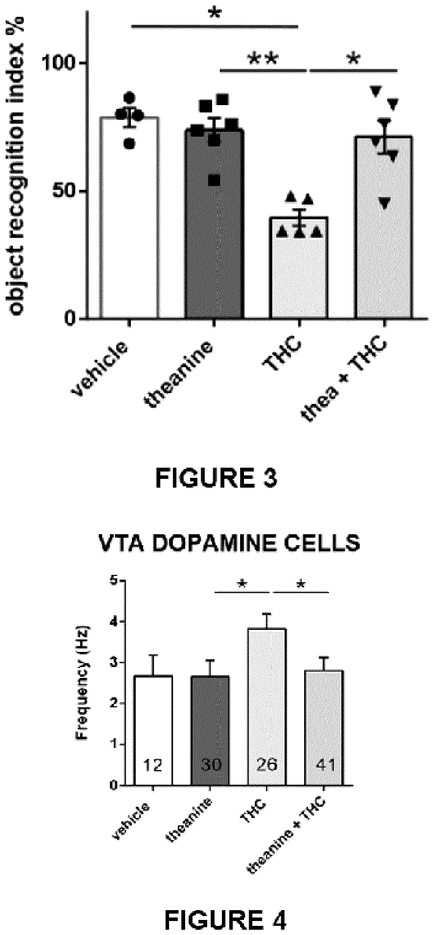

FIG. 3. Using a pre-clinical behavioural model of memory impairment (Novel Object Recognition) it was demonstrates that chronic exposure to THC caused a significant object memory impairment. This effect is blocked by co-administration with L-Theanine (10 mg/kg).
FIG. 4. Chronic THC exposure caused a significant dysregulation of the dopamine neurotransmitter system, shown by hyperactive activity rates in recordings of single dopamine neurons in the ventral tegmental area of the rat. Co-administration of THC with L-Theanine (10 mg/kg) blocks this effect.

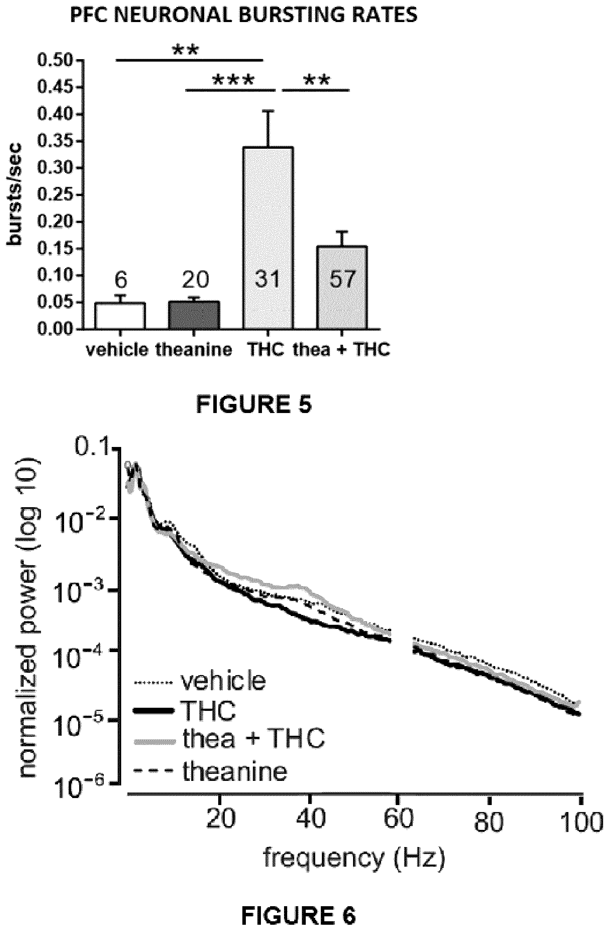

FIG. 5. Chronic THC exposure caused a significant dysregulation of the frontal cortex in rats demonstrated by a hyperactive 'bursting' pattern activity state recorded in single neurons of the prefrontal cortex (PFC). Co-administration of THC with L-Theanine (10 mg/kg) blocks this effect.
FIG. 6. Electrophysiological recordings of neural, local field potentials in the rat prefrontal cortex revealed a significant decrease in gamma-band activity states following chronic THC exposure; a neuronal biomarker of schizophrenia and other neuropsychiatric disorders. This effect was reversed by co-administration of L-Theanine (10 mg/kg).

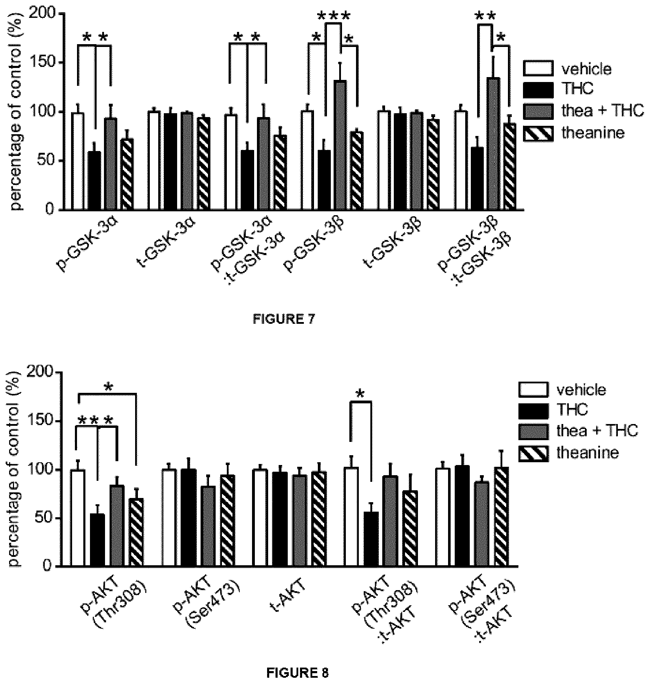

FIG. 7. Consistent with clinical findings in post-mortem schizophrenia frontal cortex tissue, chronic THC exposure caused a significant loss in the protein expression levels of glycogen-synthase-kinase-3A and 3B, both of which are linked to dopamine and prefrontal cortex neuronal dysregulation. These molecular signaling effects are prevented by co-administration with L-Theanine (10 mg/kg), as measured in a Western Blot procedure.
FIG. 8. Consistent with clinical genetic findings in human populations showing that polymorphic abnormalities in the gene encoding Akt (protein kinase B) increase vulnerability to cannabis-induced neuropsychiatric side-effects, specifically at the threanine308 binding residue, chronic THC causes significant downregulation in protein expression levels of Akt308 in the prefrontal cortex of rat brain. This effect is prevented by L-Theanine (10 mg/kg) co-administration. In contrast, no effect on Akt at serine 473 was observed, suggesting that L-Theanine can selectively prevent the expression of THC-neuropsychiatric molecular biomarkers, such as Akt308.

### DETAILED DESCRIPTION

Described is L-Theanine for use in a method of treatment according to claim 1. Said method of treatment may involve a plurality of THC administrations, the method comprising the administration of L-Theanine with each administration of THC, the administration being such that the peak bioavailability of THC occurs substantially contemporaneously with the peak bioavailability of L-Theanine. This THC exposure regimen is designed to mimic a long-term, chronic exposure period to cannabis use in humans. The dose of THC is gradually increased to account for possible receptor tolerance effects from THC exposure, as would be predicted to occur in long-term human users of cannabis.

### EXPERIMENTAL

Four groups of ten rats were studied. The first group of rats was given twice daily intra-peritoneal doses of THC. The dosage regime was as follows

| | Morning | Afternoon |
|---|---|---|
| Day 1 | 2.5 mg/kg | 2.5 mg/kg |
| Day 2 | 2.5 mg/kg | 2.5 mg/kg |
| Day 3 | 2.5 mg/kg | 2.5 mg/kg |
| Day 4 | 5 mg/kg | 5 mg/kg |
| Day 5 | 5 mg/kg | 5 mg/kg |
| Day 6 | 5 mg/kg | 5 mg/kg |
| Day 7 | 5 mg/kg | 5 mg/kg |
| Day 8 | 10 mg/kg | 10 mg/kg |
| Day 9 | 10 mg/kg | 10 mg/kg |
| Day 10 | 10 mg/kg | 10 mg/kg |

Immediately preceding each dose of THC, each rat was dosed with 10 mg/kg L-Theanine, administered intra-peritoneally.

The second group of rats was given the same doses of THC and no L-Theanine.

The third group of rats was given twice daily doses of L-Theanine but no THC.

The fourth group of rats was given neither THC nor L-Theanine.

All groups of rats were fed, watered and otherwise housed identically. At the end of the 10 day period, the rats were subject to a serious of tests.

### (Reference) Paired-pulse inhibition test for cognitive filtering deficits

Rats were acclimatized to the startle chambers (Med Associates) for 5 min over 3 days. On the last day of acclimation, rats were tested in an input/output (I/O) function consisting of 12 increasing startle pulses (from 65 to 120 dB, 5 dB increments) to determine the appropriate gain setting for each individual rat. The testing procedure consisted of the following phases: the acclimation phase, a habituation phase (Block 1), and PPI measurement (Block 2). During acclimation, rats were exposed to the chambers and white background noise (68 dB) for 5 min. During Block 1, 10 pulse alone trials (110 dB white noise, 20 ms duration) were delivered at 15-20 s inter-trial intervals. Block 2 consisted of 9 different trials presented 10 times in a pseudo-randomized order at 15-20 s intervals: 10 pulse-alone trials, and 10 of each of the three different prepulse-pulse trial types (72, 76, 80) with interstimulus intervals of 30 and 100 ms. Pulse-alone trials consisted of a startle stimulus-only presentation, whereas prepulse-pulse trials consisted of the presentation of a weaker non-startling prepulse (white noise, 20 ms duration) before the startling stimulus. PPI was calculated for each animal and each trial condition as PPI (%) = (1 - average startle amplitude to pulse with prepulse/average startle amplitude to pulse only) × 100.

### Sucrose preference testing for depression/anhedonia symptoms

Rats were water-restricted for 12 hrs prior to being given a 2-bottle choice test between a solution containing 2% sucrose vs. plain water. The amounts consumed from each bottle were measured over 24 hr for 4 days. A sucrose preference index was calculated for each rat and averaged across groups by taking the percentage of the volume of sucrose intake over the total volume of fluid intake over the 60-min test phase.

### (Reference) Novel Object Memory Test for memory deficits:

The test sessions consisted of two 3-min trials. During the first trial (T1 acquisition trial), each rat was placed in the center of an arena containing two identical objects placed in the far corners 15cm from the side wall. After a delay of 60 min during which the rat was returned to its cage, and both objects were replaced (one by an identical copy, the other by a novel object in the same location), the rat was returned to the arena for the second trial (T2 test trial). Between rats, both the role (familiar or novel object) and the relative position of the two objects were randomly counterbalanced. Object exploration was considered when the head of the rat was facing the object or the rat was touching or sniffing the object. Times spent in exploration were videotaped with a video-tracking system (ANY-maze; Stoelting) and analyzed by an experimenter blind to the treatment conditions. Exploration times were recorded and used to calculate object recognition index [time spent with novel object/total time exploring both objects]

### (Reference) Neuronal electrophysiology recordings in the VTA and PFC:

Extracellular single-unit electrophysiological recordings were performed in vivo in adult (>PND 75) pretreated rats during adolescence. The recordings were taken from either putative glutamatergic (GLUT) PFC neurons or dopaminergic (DA) VTA neurons. Rats were anesthetized with urethane (1.4g/kg, i.p., Sigma-Aldrich) and placed in a stereotaxic frame with body temperature maintained at 37 °C. A scalp incision was made and a hole was drilled in the skull overlaying the targeted structure at the following coordinates: mPFC: AP: +2.7 to +3.5 mm from bregma, L: ±0.8 to ±1 mm, DV: -2.5 to -4 mm from the dural surface; VTA: AP: -5.2 mm from bregma, L: ±0.8 to ±1 mm, DV: -6.5 to -9 mm from the dural surface. Recordings were taken with glass microelectrodes (average impedance of 6-10 MΩ) filled with 2M sodium acetate solution containing 2% pontamine sky blue (Sigma-Aldrich). A bone screw was placed over the cerebellum and was connected with the return of the headstage and served as a reference electrode. Extracellular signals were amplified (×5000) using a MultiClamp 700B amplifier (Molecular Devices), digitized at 25 kHz and recorded on the computer using a Digidata 1440 A and pClamp software (Molecular Devices). The wideband signal of PFC recordings was fed to two channels of the digitizer and filtered to obtain single unit recordings (band pass between 0.3 and 3 kHz).

**VTA DA neurons** were identified according to well established electrophysiological features86: (1) action potential width > 2.5 ms, (2) spontaneous firing rate between 2-5 Hz, (3) a triphasic waveform consisting of a notch on the rising phase followed by a delayed after potential, and (4) a single irregular or burst firing pattern.

**Putative mPFC pyramidal cells** were identified based on established criteria85: (1) firing frequency < 10 Hz, (2) waveform shape, and (3) action potential duration > 2.5 ms. Cells exhibiting 3 consecutive spikes with inter-spike intervals < 45 ms were classified as burst-firing cells85. The electrophysiological properties of spontaneously active pyramidal neurons were sampled in the mPFC by making vertical passes of the electrode through the pyramidal cell body region. These tracks were made in a predefined pattern, with each track separated by 200 µm. After an individual putative pyramidal neuron was isolated, its spontaneous activity was recorded for 5 min. Two parameters of activity were sampled, the basal firing rate and the bursting rate.

The results of the tests were as follows.

### Paired Pulse

As shown in Fig. 1:
o THC exposure alone produces strong deficits in the prepulse inhibition at both 30 and 100 msec durations between the 'pre-pulse' and test pulse.
o L-Theanine administration alone has no apparent effects on sensory filtering, relative to vehicle control rats
o THC co-administration with L-Theanine produces no apparent deficits and thereby blocked the negative effects of THC on cognitive filtering.

Thus, L-Theanine appears to block the ability of THC to cause cognitive filtering deficits.

### Sucrose Preference

As shown in Fig. 2:
o THC exposure induces strong sucrose preference reduction
o L-Theanine administration alone has no apparent effects on sucrose preference, relative to vehicle control rats
o THC co-administration with L-Theanine significantly reduces sucrose preferences compared to THC rats.

Thus, L-Theanine appears to block the ability of THC to induce anhedonic, depressive-like effects.

### Novel Object Testing

As shown in Fig. 3:
o THC exposure induces strong reductions in object recognition
o L-Theanine administration alone has no apparent effects on object recognition
o THC co-administration with L-Theanine significantly improves object recognition compared to THC rats.

Thus, L-Theanine appears to prevent short term memory deficits associated with THC exposure, with no apparent effects on short-term memory processing in and of itself.

### VTA Testing

As shown in FIG. 4, L-Theanine with THC co-administration prevents neuronal hyperactivity recorded in dopamine neurons in the mesolimbic pathway

### PFC Neuronal Burst Testing

As shown in FIG. 5, L-Theanine administration with THC can block the neuronal hyperactivity observed in the brain's prefrontal cortex induced by THC exposure.

### Theory

### Normalized Power Testing

As shown in FIG. 6, L-Theanine + THC rats demonstrated generally enhanced gamma wave activity through the 0-100 Hz range, in contrast to generally decreased activity on the part of the THC only rats; given that schizophrenia is associated with lower gamma wave activity over this range, the enhanced activity associated with L-Theanine is suggestive of improvement in terms of reduced schizophrenic side effects in the context of THC use.

### GSK-3a and GSK- 3β

As shown in FIG. 7, whereas THC-alone resulted in significant decrease in the levels of the GSK-3a and GSK- 3β, the co-administration of L-Theanine appeared to reverse this effect; given that these two isoforms are strongly related to schizophrenia, the tests suggests that co-administration of L-Theanine will reduce the schizophrenia-like symptoms sometimes associated with chronic THC use.

### Thr308

The Thr308 gene is linked to susceptibility to long-term cannabis-related psychotic effects. The co-administration of L-Theanine appeared to counteract the effects of THC, as indicated by FIG. 8. This strongly augurs in favor of co-administration of L-Theanine in the context of any THC treatment of persons having this gene.

### About the Commentary

In this document, use is made of the terms "reverse", "block", "prevent", "counteract", "improve", such terms should be interpreted reasonably, with reference to the experimental results; thus, for example, "prevent" should not necessarily be construed to require a complete prevention in all cases.

### About the Dosages

Whereas in this document, specific dosage regimes are described, it will be appreciated by persons of ordinary skill that the dosages mentioned, which have been proven to be useful in the context of rats and in experimental conditions selected to mimic/approximate chronic THC exposure in humans; routine experimentation will be required to modify the dosages for human use.

### Variations

Whereas the experimental evidence relates to substantially contemporaneous intra-peritoneal administration, it is reasonable to assume that any other method of administration wherein the peak bioavailability of THC occurs substantially contemporaneously with the peak bioavailability of L-Theanine will have similar utility.

Thus, for example and without limitation, persons of ordinary skill could be expected to produce consumer packages adapted to carry out the method in at least the following forms: intravenous bag, prefilled syringe, cream, patch, pill, capsule, liquid, inhaler, sublingual strip and suppository.

Techniques such as the DehydraTECH^{™} system of Lexaria Bioscience Corp. would also be expected to be useful to produce edibles. The NANOSTABILIZER^{™} of Industrial Sonomechanics would also be expected to be useful in formulation.

## Claims

1. L-Theanine for use in a method of treatment for blocking THC-induced anhedonic, depressive-like effects, wherein the treatment regime includes the exposure of a patient to Δ-9-Tetrahydrocannabinol (THC).

2. Theanine for use in the therapeutic method of claim 1,wherein the treatment regime involves a plurality of administrations of Δ-9-Tetrahydrocannabinol (THC), and wherein the method comprises the step of administering Theanine to the patient with each administration of Δ-9-Tetrahydrocannabinol (THC).

3. Theanine for use in the therapeutic method of claim 2, wherein the method of administration is such that the peak bioavailability of Δ-9-Tetrahydrocannabinol (THC) occurs substantially contemporaneously with the peak bioavailability of Theanine.

4. Theanine for use in the therapeutic method of claim 3, wherein the administration is by needle, transdermal, ingestion, inhalation, sublingual or by suppository.

## Patentansprüche

1. L-Theanin zur Verwendung in einem Behandlungsverfahren zur Blockierung von THC-induzierten anhedonischen, depressionsähnlichen Wirkungen, wobei das Behandlungsregime die Exposition eines Patienten gegenüber Δ-9-Tetrahydrocannabinol (THC) umfasst.

2. Theanin zur Verwendung in dem therapeutischen Verfahren gemäß Anspruch 1, wobei das Behandlungsregime eine Vielzahl von Verabreichungen von Δ-9-Tetrahydrocannabinol (THC) umfasst und wobei das Verfahren den Schritt der Verabreichung von Theanin an den Patienten mit jeder Verabreichung von Δ-9-Tetrahydrocannabinol (THC) umfasst.

3. Theanin zur Verwendung in dem therapeutischen Verfahren gemäß Anspruch 2, wobei das Verabreichungsverfahren so beschaffen ist, dass die maximale Bioverfügbarkeit von Δ-9-Tetrahydrocannabinol (THC) im Wesentlichen zeitgleich mit der maximalen Bioverfügbarkeit von Theanin auftritt.

4. Theanin zur Verwendung in dem therapeutischen Verfahren gemäß Anspruch 3, wobei die Verabreichung über eine Nadel, transdermal, durch Einnahme, Inhalation, sublingual oder als Zäpfchen erfolgt.

## Revendications

1. L-théanine destinée à être utilisée dans un procédé de traitement visant à bloquer les effets anhédoniques, de type dépressif, induits par le THC, le schéma thérapeutique incluant l'exposition d'un patient au Δ-9-tétrahydrocannabinol (THC).

2. Théanine destinée à être utilisée dans le procédé thérapeutique de la revendication 1, le schéma thérapeutique comprenant une pluralité d'administrations de Δ-9-tétrahydrocannabinol (THC), et le procédé comprenant l'étape consistant à administrer de la théanine au patient avec chaque administration de Δ-9-tétra-hydrocannabinol (THC).

3. Théanine destinée à être utilisée dans le procédé thérapeutique de la revendication 2, le mode d'administration étant tel que le pic de biodisponibilité du Δ-9-tétrahydrocannabinol (THC) apparaît pratiquement en même temps que le pic de biodisponibilité de la théanine.

4. Théanine destinée à être utilisée dans le procédé thérapeutique de la revendication 3, l'administration étant par injection, transdermique, par ingestion, par inhalation, sublinguale ou par suppositoire.
